# EUROPEAN PATENT APPLICATION

(11) **EP 4 484 551 A1**
(43) Date of publication of application: **01.01.2025**
(21) Application number: 22928058.1
(22) Date of filing: 26.05.2022
(51) Int. Cl.: C12N 1/21, C12N 15/31, C12N 15/74, C12N 15/113, C07K 14/47, A61K 39/04, A61P 31/06, C12R 1/32

(54) **USE OF BCG GENE BCG_1820 IN PREPARING TUBERCULOSIS VACCINE RECOMBINANT BCG**

(30) Priority: 24.02.2022 CN 202210178167
(71) Applicant: Shanghai Pulmonary Hospital, Shanghai 200433 (CN)
(72) Inventor: GE, Baoxue, Shanghai 200433 (CN); WANG, Lin, Shanghai 200433 (CN); PENG, Cheng, Shanghai 200433 (CN); YANG, Hua, Shanghai 200433 (CN); WANG, Jie, Shanghai 200433 (CN)
(74) Representative: Cabinet Chaillot
(86) International application number: PCT/CN2022/095396
(87) International publication number: WO 2023/159787

(57) **Abstract**

Provided are a BCG recombinant strain ΔBCG_1820 in which BCG_1820 gene is knocked out, a preparation method therefor, and the use thereof in preparing a tuberculosis vaccine. The BCG recombinant strain ΔBCG_1820 can induce macrophages to produce more antibacterial peptides, so as to endow a host with a stronger capability to resist tubercle bacillus infection, and has the potential to be a candidate vaccine for tubercle bacillus.

## Description

### BACKGROUND OF THE INVENTION

### 1. Field of the Invention

The present invention relates to the field of biomedicine, in particular to the use of BCG gene *BCG_1820* in preparing tuberculosis vaccine recombinant BCG.

### 2. Description of the Related Art

Tuberculosis is the leading cause of death from bacterial infectious diseases worldwide. The global tuberculosis situation remains critical due to the lack of an effective preventive vaccine against tuberculosis in adults. The only vaccine currently licensed for clinical use to prevent tuberculosis is Mycobacterium bovisbacille Calmette-Guérin (BCG), a live attenuated vaccine. BCG was first injected into newborns in Paris in 1921¹. By 2019, 88% of children globally received BCG vaccination in their first year of life². BCG has more than 70% protection against tuberculous meningitis in neonates and school-age children³. However, protection against tuberculosis in adults is very limited. At the beginning of the 21st century, the research and development of new tuberculosis vaccines has been increasing around the world. It includes subunit adjuvant formulations of Mtb fusion proteins, viral vector vaccines expressing one or more antigens of Mtb, inactivated mycobacterial vaccines, and attenuated mycobacterial vaccines^{4,5}.

The development of a new tuberculosis vaccine follows two basic ways^{6,7}. The first way is to replace BCG with a modified recombinant BCG (rBCG) or knockout-attenuated Mycobacterium tuberculosis. Genetically modified rBCG should be characterized by: a) being safer; b) stronger immunogenicity; c) inducing more durable protection; d) protective effect against highly virulent clinical isolates, such as Mycobacterium tuberculosis Beijing strain, multidrug-resistant strain (MDR), extensively drug-resistant tuberculosis strain (XDR), etc. One way to make recombinant BCG is to introduce immunogenic TB-specific antigens that are lacking in BCG, such as the tuberculosis antigen gene encoded by RD1 (ESAT6, CFP10); or by overexpression of BCG autoantigens (homologs of the Ag85 complex, etc.). Another method of recombinant BCG is gene editing of existing BCG to better enhance the host innate immune response⁸. In addition to these two rBCG vaccine approaches, another strategy for developing a tuberculosis vaccine is attenuation of Mycobacterium tuberculosis. This includes the deletion of essential metabolic genes to produce auxotrophic mutants, or the major deletion of virulence genes and their regulators. One study showed that expressing the RD1 antigen of Mycobacterium tuberculosis on Mycobacterium voles significantly improved host resistance to tuberculosis infection⁹. Recombinant Mycobacterium smegmatis has also been found to be a vaccine for tuberculosis. When Mycobacterium smegmatis knocks out the *ESX-3* gene, a strong innate immune response can be seen in the immunized mice. When this recombinant Mycobacterium smegmatis was transferred back to the *ESX-3* gene of Mycobacterium tuberculosis, better protective activity against the host was observed in a mouse model of challenge against Mycobacterium tuberculosis.

The second major way for developing tuberculosis vaccines is to construct subunit vaccines. These vaccines are either non-live vaccines, or non-replicating vaccines that use the virus as a vector. Subunit vaccines for tuberculosis are mainly recombinant proteins, or use attenuated viral vectors. While subunit vaccines can theoretically be used as a start-up vaccine, the prevailing view is that they can only be used as booster vaccines on top of adjuvant BCG, recombinant BCG, or attenuated Mtb vaccines.

At present, BCG has limited protective effect on pulmonary tuberculosis patients, and the construction of recombinant BCG is the main research direction. The main research strategy of recombinant BCG is to knock out the virulence gene of BCG, activate the immune response function of the host, and improve the protective effect of the existing vaccine BCG. However, it is not clear which genes are important immunosuppressive factors in BCG, and there is still a lack of theoretical basis for gene editing of BCG based on which target can improve the protective effect of BCG.

Antimicrobial peptides (AMPs) are considered to be the ancient defensive weapon of the innate immune system of organisms, with a wide range of activity against Gram-positive and Gram-negative bacteria, fungi, parasites and viruses. AMPs typically consist of 12-15 amino acids with cations (composed of positively charged arginine and lysine residues)¹⁰ and act by interacting with negatively charged bacterial membranes, resulting in phospholipid displacement, membrane structural disorders, and internalization¹¹. Because of the different mechanisms of action of AMPs, microorganisms rarely develop resistance. Based on the host's antimicrobial peptide, the present invention is to find the BCG virulence gene that significantly inhibits its expression, so as to provide a strategy for constructing a recombinant BCG vaccine with better effect.

### SUMMARY OF THE INVENTION

In order to overcome the defects in the prior art, the present invention finds a recombinant BCG strain constructed by missing virulence genes *BCG_1820* on wild-type BCG strain to significantly improve the immune protection effect of BCG and provides candidates for the development of tuberculosis vaccine. Based on this, the present invention provides the use of BCG gene *BCG_1820* in preparing tuberculosis vaccine recombinant BCG.

In order to achieve the above purpose, the present invention adopts the following technical solution.

On the first aspect of the invention, there is provided a ΔBCG_1820 bacteria, *BCG_1820* gene in the BCG recombinant bacteria is knocked out.

Further, the *BCG_1820* gene in the BCG recombinant bacteria is knocked out through CRISPR/Cas9 technology.

Further, in the knockout process, gRNA sequence of the *BCG_1820* gene, which is adopted, is SEQ ID No.: 2.

On the second aspect of the invention, there is provided a construction method of the BCG recombinant bacteria, in which the BCG recombinant bacteria is obtained by knocking out the *BCG_1820* gene in a wild-type BCG strain through CRISPR/Cas9 technology.

On the third aspect of the invention, there is provided a use of the BCG recombinant bacteria in preparing tuberculosis vaccine, wherein the tuberculosis vaccine comprises the BCG recombinant bacteria.

Further, the tuberculosis vaccine further comprises an adjuvant.

On the fourth aspect of the invention, there is provided a tuberculosis vaccine recombinant BCG, wherein the tuberculosis vaccine recombinant BCG is a BCG in which the *BCG_1820* gene is knocked out.

Further, the *BCG_1820* gene is knocked out through CRISPR/Cas9 technology.

Further, in the knockout process, gRNA sequence of the *BCG_1820* gene, which is adopted, is SEQ ID No.: 2.

On the fifth aspect of the invention, there is provided a BCG strain *BCG_1820* gene knockout vector, wherein the knockout vector is a gRNA expression vector based on CRISPR/Cas9 system, the gRNA sequence is SEQ ID No.: 2.

On the sixth aspect of the invention, there is provided a use of the BCG strain *BCG_1820* gene knockout vector in preparing tuberculosis vaccine

By adopting the above technical solution, in comparison with the prior art, the invention has the following technical effects:
The BCG recombinant strain ΔBCG_1820 can induce macrophages to produce more antibacterial peptides, so as to endow a host with a stronger capability to resist tubercle bacillus infection, and has the potential to be a candidate vaccine for tubercle bacillus.

### BRIEF DESCRIPTION OF THE DRAWINGS

Fig. 1 shows the result of PCR identification of *BCG_1820* gene knockout strain in an embodiment of the present invention;
Fig. 2 shows that the ΔBCG_1820 strain in an embodiment of the present invention, which significantly promotes the expression of macrophage antimicrobial peptide genes; wherein Figs. A-D show the effects of ΔBCG_1820 strains on the expression levels of *Camp, Hamp, Dejb3* and *Defb4* in macrophages, respectively.
Fig. 3 shows the flow chart of mouse immune challenge experiment in an embodiment of the present invention;
Fig. 4 shows the comparison results of the amount of bacterial load in lung tissue of the mouse performing challenge experiment for 30 days after 30 days of immunization in an embodiment of the present invention;
Fig. 5 shows the results of pathological HE staining (Fig. A) and acid-fast staining (Fig. B) in the lungs of immunized mouse after 30 days of infection in an embodiment of the present invention.

### DETAILED DESCRIPTION

The invention provides the use of BCG gene *BCG_1820* in preparing tuberculosis vaccine recombinant BCG. The amino acid sequence of the *BCG_1820* gene (source database: https://www.uniprot.org/uniprot/A0A0H3M6W4) is SEQ ID No.:1.

The present invention is described in detail and specifically through specific embodiments and drawings below to enable a better understanding of the present invention, but the following embodiments do not limit the scope of the present invention.

In the embodiment, the method, if there is no special description, is a conventional method, and the reagent, if there is no special description, is a conventional commercially available reagent or the reagent prepared according to the conventional method.

### Example 1

In the example, a *BCG_1820* gene deletion strain was constructed on the wild-type BCG strain, and the specific construction process and results are as follows.

*A BCG_1820* gene deletion strain (ΔBCG_1820) was constructed on wild-type BCG Denmark strains using Cas9 technology.

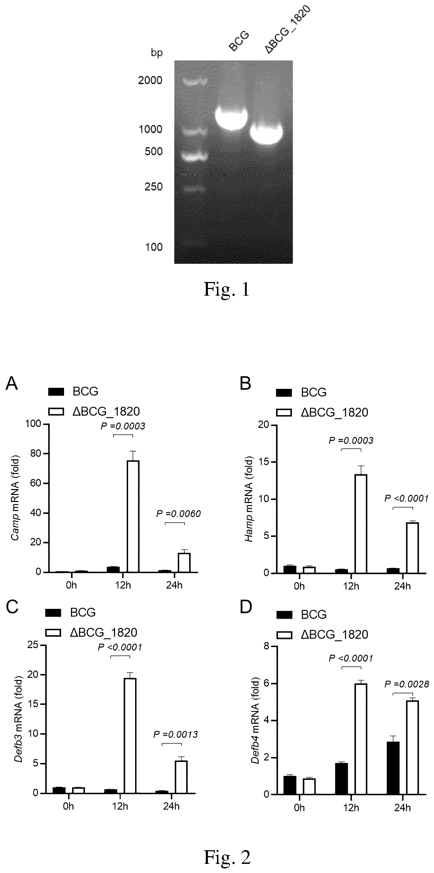

First, BCG:pYC1759 competent cells were prepared, the pYC1759 plasmid was electro-transferred to the wild-type BCG Denmark strain, the BCG monoclonal strain, which was successfully transferred to the plasmid, was picked, and was performed by amplification of bacteria in K+, 7H9+OADC medium, followed by glycerol washing for three times, and the competent cells were collected at -80 degrees cryopreservation for later use. The sgRNA expression plasmids of Cas9 and *BCG_1820* genes (the gRNA sequence of the *BCG_1820* gene was ATCGGCTCCGCATTGAACGC (SEQ ID No.: 2)) were electro-transferred in BCG:pYC1759 competent cells, and were amplified and coated on K+Zeo resistant culture plates, and the monoclonal was picked for PCR and sequencing identification, and the results are shown in Fig. 1.

### Example 2

In this embodiment, on the basis of Example 1, it is verified that the ΔBCG_1820 strain can induce macrophages to produce more antimicrobial peptides, and the specific experimental procedures and results are as follows:
Cells were lysed by Trizol after infection of wild-type BCG strain and ΔBCG_1820 strain (MOI=5) for 12 hours and 24 hours using a mouse peritoneal primary macrophage infection model, and total RNA was extracted, and was reverse transcribed into cDNA, and then was performed by quantitative analysis for *Camp, Hamp, Dejb3* and *Defb4* in cells by QPCR.

As shown in Fig. 2, the BCG with the *BCG_1820* gene knocked out significantly promotes the expression of antimicrobial peptides, suggesting that the protein encoded by the *BCG_1820* gene can inhibit the expression of host antimicrobial peptides and is a virulence factor of BCG.

### Example 3

In this embodiment, it is verified that at the animal level, the ΔBCG_1820 strain has stronger immunoprotective function than the BCG strain, and the specific experimental procedures and results are as follows.

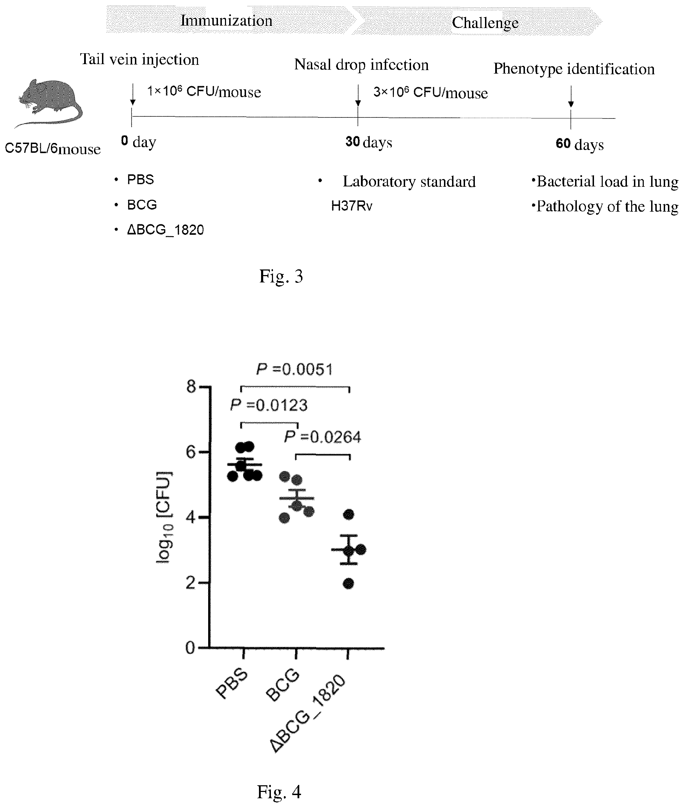

Referring to the flowchart in Fig. 3, wild-type C57BL/6 mice were given tail vein injections with PBS, 1×10⁶ CFU BCG strains, or 1×10⁶ CFU ΔBCG_1820 strains, respectively. After 30 days of immunization, mice in each group were administered to the respiratory tract infection of tuberculosis strain H37Rv in the biosafety level 3 laboratory. After 30 days of infection with tuberculosis bacteria, the mice were sacrificed with neck dislocation, and the lung tissues were isolated for CFU counting, and the amount of bacteria load in the lung tissues of the mice in each group was confirmed, and the lung tissues of the mice in each group were fixed with 4% PFA, and the pathological changes of the lung tissues between the groups were observed by paraffin embedding, tissue sections and H&E staining.

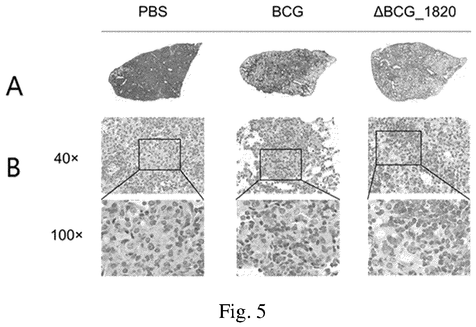

As shown in Fig. 4, the ΔBCG_1820 strain has a 30-fold decrease amount of bacteria load in lung tissue compared to the immunized wild-type BCG strain, with less neutrophil infiltration and more intact alveolar tissue (Fig. 5).

In conclusion, the ΔBCG_1820 strain can better protect the host against tuberculosis infection than the BCG strain.

The specific embodiments of the present invention are described in detail above, but they are only used as examples, and the present invention is not limited to the specific embodiments described above. For those skilled in the art, any equivalent modification and substitution of the present invention is also within the scope of the present invention. Therefore, equal transformations and modifications made without departing from the spirit and scope of the present invention shall be covered within the scope of the present invention.

### Reference Documents

1. Tidjani, O., Grunitzky, B., Sadjo, H. & Guérin, N. [The prophylaxis of tuberculosis and vaccination with BCG. A recent study]. Ann Pediatr (Paris)39 (1992).
2. Chard, A. N., Gacic-Dobo, M., Diallo, M. S., Sodha, S. V. & Wallace, A. S. Routine Vaccination Coverage - Worldwide, 2019. MMWR Morb Mortal Wkly Rep69, 1706-1710, doi:10.15585/mmwr.mm6945a7 (2020).
3. Mangtani, P. et al. Protection by BCG vaccine against tuberculosis: a systematic review of randomized controlled trials. Clin Infect Dis58, 470-480, doi:10.1093/cid/cit790 (2014).
4. Ginsberg, A. M. Designing tuberculosis vaccine efficacy trials - lessons from recent studies. Expert Rev vaccines18, 423-432, doi:10.1080/14760584.2019.1593143 (2019).
5. Ottenhoff, T. H. M. & Kaufmann, S. H. E. Vaccines against tuberculosis: where are we and where do we need to go? PLoS Pathog8, e1002607, doi: 10.1371 /j ournal. ppat.1 002607 (2012).
6. Kaufmann, S. H. E. Future vaccination strategies against tuberculosis: thinking outside the box. Immunity33, 567-577, doi:10.1016/j.immuni.2010.09.015 (2010).
7. Ottenhoff, T. H. M. Overcoming the global crisis: "yes, we can", but also for TB ... ? Eur J Immunol39, 2014-2020, doi:10.1002/eji.200939518 (2009).
8. Reece, S. T. & Kaufmann, S. H. E. Floating between the poles of pathology and protection: can we pin down the granuloma in tuberculosis? Curr Opin Microbiol15, 63-70, doi:10.1016/j.mib.2011.10.006 (2012).
9. Brodin, P. et al. Enhanced protection against tuberculosis by vaccination with recombinant Mycobacterium microti vaccine that induces T cell immunity against region of difference 1 antigens. J Infect Dis190, 115-122 (2004).
10. Hancock, R. E. & Lehrer, R. Cationic peptides: a new source of antibiotics. Trends Biotechnol16, 82-88 (1998).
11. Lakshmaiah Narayana, J. & Chen, J.-Y. Antimicrobial peptides: Possible anti-infective agents. Peptides72, 88-94, doi:10.1016/j.peptides.2015.05.012 (2015).

## Claims

1. A BCG recombinant bacteria, wherein the BCG recombinant bacteria is a ΔBCG_1820 strain, *BCG_1820* gene in the BCG recombinant bacteria is knocked out.

2. The BCG recombinant bacteria of claim 1, wherein the *BCG_1820* gene in the BCG recombinant bacteria is knocked out through CRISPR/Cas9 technology.

3. The BCG recombinant bacteria of claim 2, wherein in the knockout process, gRNA sequence of the *BCG_1820* gene, which is adopted, is SEQ ID No.: 2.

4. The construction method of BCG recombinant bacteria of any one of claims 1-3, wherein the BCG recombinant bacteria is obtained by knocking out the *BCG_1820* gene in a wild-type BCG strain via CRISPR/Cas9 technology.

5. The use of the BCG recombinant bacteria of any one of claims 1-3 in preparing tuberculosis vaccine, wherein the tuberculosis vaccine comprises the BCG recombinant bacteria.

6. The use of claim 6, wherein the tuberculosis vaccine further comprises an adjuvant.

7. A tuberculosis vaccine recombinant BCG, wherein the tuberculosis vaccine recombinant BCG is a BCG in which the *BCG_1820* gene is knocked out.

8. The tuberculosis vaccine recombinant BCG of claim 7, wherein the *BCG_1820* gene is knocked out by CRISPR/Cas9 technology; in the knockout process, preferably, gRNA sequence of the *BCG_1820* gene, which is adopted, is SEQ ID No.: 2.

9. A BCG strain *BCG_1820* gene knockout vector, wherein the knockout vector is a gRNA expression vector based on CRISPR/Cas9 system, the gRNA sequence is SEQ ID No.: 2.

10. The use of the BCG strain *BCG_1820* gene knockout vector of claim 9 in preparing tuberculosis vaccine.
